# EUROPÄISCHE PATENTSCHRIFT

(11) **EP 0 966 045 B1**
(45) Veröffentlichungstag und Bekanntmachung des Hinweises auf die Patenterteilung: **17.06.2009**
(21) Anmeldenummer: 98111318.6
(22) Anmeldetag: 19.06.1998
(51) Int. Cl.: H01L 29/49, H01L 23/532

(54) **Verfahren zum Herstellen einer Metallelektrode in einer Halbleiteranordnung mit einem MOS-Transistor**
Method of manufacturing a metal electrode in a semiconductor device having a MOS transistor
Procédé de fabrication d'une électrode métallique dans un dispositif semi-conducteur avec un transistor MOS

(43) Veröffentlichungstag der Anmeldung: 22.12.1999
(73) Patentinhaber: Micronas GmbH, 79108 Freiburg i. Br. (DE)
(72) Erfinder: Igel, Günter, Dipl.-Ing., 79331 Teningen (DE)
(74) Vertreter: Patentanwälte Westphal, Mussgnug & Partner

(56) Entgegenhaltungen:
- EP-A- 0 121 605
- EP-A- 0 607 732
- EP-A- 0 841 561
- WO-A-81/00304
- GB-A- 2 184 288
- US-A- 3 775 192
- US-A- 4 442 137
- US-A- 4 569 743
- US-A- 4 704 304
- US-A- 4 994 154
- US-A- 5 104 820
- LUNDSTROM I ET AL: "A hydrogen-sensitive MOS field-effect transistor" APPLIED PHYSICS LETTERS, 15 JAN. 1975, USA, Bd. 26, Nr. 2, Seiten 55-57, XP002086960 ISSN 0003-6951

## Beschreibung

Die Erfindung betrifft ein Verfahren zum Herstellen einer Metallelektrode in einer Halbleiteranordnung mit einem MOS-Transistor.

Ein solches Verfahren ist z.B. EP 0 841 561 A2 bekannt. Während des MOS-Prozesses wird ein Sensorbereich für den Sensor mit einer Struktur für die Metallelektrode erzeugt, wobei die Struktur aus einem Material mit vorbestimmten Hafteigenschaften für Metalle besteht und der Sensorbereich durch Ätzen einer Passivierungsschicht der MOS-Anordnung freigelegt wird.

Bei einer Metallisierung der Oberfläche der MOS-Anordnung bleibt die Metallschicht nur auf der Struktur für die Metallelektroden haften.

Es gibt in der Halbleitertechnologie verschiedene Anwendungsmöglichkeiten, bei denen es erforderlich ist, strukturierte Metallschichten aufzubringen. Beispielsweise können Metallschichten innerhalb von vorgegebenen Bereichen auf der Oberfläche der Halbleiteranordnung erforderlich sein. Ein Anwendungsbeispiel, bei dem eine strukturierte Metallschicht aufgebracht wird, ist das Herstellen einer Metallelektrode für einen Sensor. Ein derartiger Sensor ist aus I. Lundstrom, S. Shivaraman, C. Svensson and L. Lundkvist, "Hydrogen sensitive MOS field-effect transistor", Appl. Phys. Lett. 26, 55-57 bekannt. Die Metallelektroden werden üblicherweise aus Edelmetallen, z.B. aus Palladium, Gold oder Platin hergestellt.

Das Herstellen einer solchen strukturierten Metallschicht ist in der Praxis insbesondere dann problematisch, wenn sie in einer MOS-Anordnung aufgebracht werden soll, da sich die Herstellung nicht mit dem üblichen MOS-Verfahren kombinieren läßt. Es müßten nämlich nach dem MOS-Verfahren zusätzliche Verfahrensschritte zum Aufbringen der strukturierten Metallschicht ausgeführt werden, die insbesondere Photolithographieprozesse, Ätzprozesse und Lackablöseprozesse umfassen.

Wird eine solche Metallelektrode auf eine MOS-Anordnung aufgebracht, so wird durch die hierzu erforderlichen Verfahrensschritte die Anordnung durch das Metall verunreinigt. Dadurch werden die Eigenschaften von Siliziumoxid, und bei MOS-Transistoren insbesondere die Eigenschaften des Gate-Oxids, und des darunter liegenden Kanals beeinträchtigt. Ferner werden die zum Durchführen der Verfahrensschritte verwendeten Vorrichtungen in der Halbleiterfabrik verunreinigt, was in der MOS-Fertigung nicht zulässig ist. Wird eine Metallelektrode aus einem unedlen Metall, wie Aluminium oder Titan aufgebracht, so verändern diese Metalle während ihrer Herstellung und bei Kontakt mit anderen Materialien ihre Eigenschaften. Als Folge ändern sich auch die Eigenschaften des Sensors. Bei der Verwendung von Platin als Metallelektrode ist ein Haftvermittler erforderlich, für welchen Titan verwendet wird. In diesem Fall treffen die oben beschriebenen ungünstigen Eigenschaften zusammen. Zudem wird das Verfahren aufgrund der zusätzlich durchzuführenden Verfahrensschritte zum Aufbringen der Metallelektrode aufwendig und teuer.

Der Erfindung liegt die Aufgabe zugrunde, ein wirtschaftlich rentables Verfahren zum Herstellen einer Metallelektrode in einer Halbleiteranordnung mit einem MOS-Transistor zu schaffen, das ohne Ätzschritte zur Bildung der Metallelektrode auskommt.

Diese Aufgabe wird durch ein Verfahren mit den Merkmalen des Anspruchs 1 gelöst.

Weiterbildungen der Erfindung sind Gegenstand der Unteransprüche.

Mit dem erfindungsgemäßen Verfahren wird die Metallschicht durch geeignete Auswahl der Materialeigenschaften der Metallschicht und der Strukturelemente sowie der geometrischen Anordnung, nämlich dem Abstand zwischen den Strukturelementen und der Dicke der Metallschicht aufgebracht. Wenn die Metallschicht auf eine MOS-Anordnung aufgebracht wird, so kann die MOS-Anordnung mittels dem Fachmann bekannten Prozeßschritten mit der Schicht und den Strukturelementen hergestellt werden. Erst wenn der MOS-Prozeß abgeschlossen ist, wird die Halbleiteranordnung metallisiert. Es ist hier keine Zusatzmaske erforderlich, da bei dem Metallisierungsverfahren die gesamte Oberfläche der Halbleiteranordnung behandelt wird, das Metall aber nur auf den Strukturelementen und der Schicht zwischen den Strukturelementen haften bleibt. Außerhalb der Strukturelemente bleibt die Metallschicht nicht haften. Dies wird dadurch erreicht, daß der Abstand bis zu einem weiteren Strukturelement oder einem diesem in der Funktion entsprechenden Element entsprechend gewählt ist.

Da zusätzliche Verfahrensschritte zum Herstellen der strukturierten Metallschicht eingespart werden können, ist das erfindungsgemäße Verfahren sehr einfach. Insbesondere wird dadurch aber auch die Herstellung einer Metallelektrode in Kombination mit einem MOS-Verfahren erst ermöglicht. Da zum Herstellen der strukturierten Metallschicht keine Photolithographieprozesse, Ätzprozesse oder Lackablöseprozesse erforderlich sind, kann die strukturierte Metallschicht aus Edelmetallen hergestellt werden, ohne daß die MOS-Anordnung oder die Vorrichtungen zur Durchführung des MOS-Verfahrens kontaminiert werden.

Gemäß einem bevorzugten Ausführungsbeispiel der Erfindung kann das Aufbringen der Metallschicht durch Aufdampfen eines Metalls und durch darauffolgendes selektives, materialabhängiges Entfernen der Metallschicht mittels Ultraschall erfolgen. Bei geeigneter Wahl der Dicke d, des Abstandes a und dem Material der Strukturelemente und des Metalls wird das aufgedampfte Metall außerhalb der Strukturelemente mit dem vorgegebenen Abstand a aufgrund der Ultraschallbehandlung zuverlässig und einfach entfernt und bleibt auf den Strukturelementen und zwischen den Strukturelementen zuverlässig haften. Die Haftung der Metallschicht zwischen den Strukturelementen wird trotz der schlechten Hafteigenschaften der Schicht für das Metall aufgrund der geometrischen Anordnung der Strukturelemente mit den guten Hafteigenschaften für das Metall gewährleistet.

Zum Aufbringen der strukturierten Metallschicht sind hier keine Verfahrensschritte erforderlich, die die Halbleiteranordnung, insbesondere wenn diese MOS-Elemente umfaßt, ungünstig beeinflussen können. Auch sind keine kostenaufwendige mit einer Maskierung verbundene Prozeßschritte nötig.

Es ist günstig, wenn zur Metallisierung ein Edelmetall, insbesondere Palladium verwendet wird. Ein selektives Haften des Paladiums auf den Strukturelementen und der Schicht zwischen den Strukturelementen erfolgt sehr zuverlässig. Es ist vorteilhaft, die Metallschicht vor dem selektiven Entfernen mit einem wasserstoffhaltigen Gas zu behandeln. Dann erfolgt die selektive Entfernung der Metallschicht zuverlässiger und schneller.

Gemäß einem anderen Ausführungsbeispiel der Erfindung erfolgt das Aufbringen der Metallschicht durch galvanisches, insbesondere durch stromloses galvanisches Abscheiden eines Metalls. Das Material der Strukturelemente und das Metall müssen dabei geeignet gewählt werden. Zwischen den Strukturelementen bleibt das Metall aufgrund der geometrischen Anordnung haften. Bei der stromlosen galvanischen Abscheidung des Metalls muß das abzuscheidende Metall edler sein als das Material der Strukturelemente.

Gemäß der Erfindung wird auf der Halbleiteranordnung ein MOS-Transistor ausgebildet, die Schicht als dessen Gate-Isolatorschicht aufgebracht, auf welcher beispielsweise eine Polysiliziumschicht derart ausgebildet wird, daß sie den Strukturelementen entsprechende Gate-Elektroden mit einem Abstand a zueinander bildet. Der MOS-Transistor kann als Sensor verwendet werden. Dabei können die Gate-Isolatorschicht aus Siliziumoxid, die Strukturelemente aus Polysilizium und die als Gate dienende strukturierte Metallschicht aus Palladium gebildet sein.

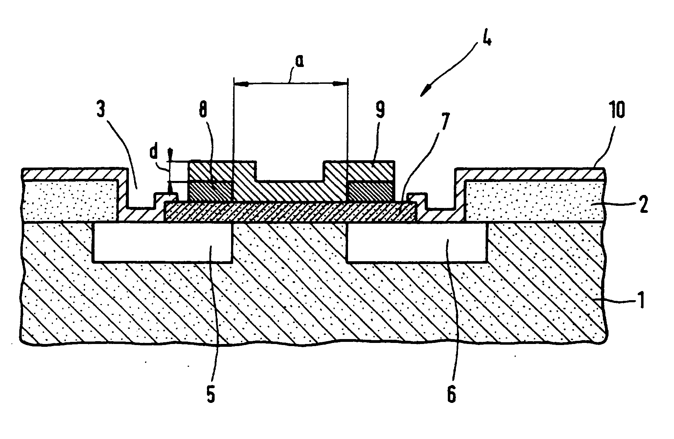

Im folgenden wird die Erfindung anhand der Zeichnung näher erläutert.

In der Figur ist ein Ausführungsbeispiel einer nach dem erfindungsgemäßen Verfahren hergestellten Vorrichtung gezeigt. Die gezeigte Halbleiteranordnung stellt einen Sensor in der MOS-Technologie dar. Auf einem Halbleitersubstrat 1 ist eine Feldoxidschicht 2 aufgebracht. In einer Öffnung 3 der Feldoxidschicht 2 ist ein als Sensor wirkender MOS-Transistor 4 ausgebildet. Der MOS-Transistor 4 umfaßt einen Drain-Bereich 5 und einen Source-Bereich 6 sowie einen Gate-Bereich. Letzterer weist eine Schicht 7, welche eine Isolatorschicht ist, auf. Auf der Schicht 7 sind Strukturelemente 8 aufgebracht, welche Gate-Elektroden des Transistors bilden und einen Abstand a zueinander haben. Auf den Strukturelementen 8 und der Schicht 7 zwischen den Strukturelementen 8 ist eine Metallschicht 9 mit der Dicke d aufgebracht. Das Material der Schicht 7 hat schlechte Hafteigenschaften für das Metall der Metallschicht 9 und das Material der Strukturelemente 8 hat gute Hafteigenschaften für das Metall der Metallschicht 9. Beispielsweise können die Schicht 7 aus Siliziumoxid, die Strukturelemente 8 aus Polysilizium und die Metallschicht 9 aus Palladium bestehen. Zur Kontaktierung des Drain-Bereiches 5 und des Source-Bereiches 6 sind Kontaktleitungen 10 aufgebracht.

Das Verhältnis des Abstandes a zwischen den Strukturelementen 8 zu der Dicke d der Metallschicht 9 ist größer als 5, und der Abstand a zwischen den Strukturelementen 8 ist kleiner als 10 µm. Die Haftung zwischen der Metallschicht 9 und den Strukturelementen 8 erfolgt aufgrund der gewählten Materialeigenschaften, die Haftung der Metallschicht 9 auf der Schicht 7 zwischen den Strukturelementen 8 aufgrund der gewählten geometrischen Bedingungen und der Haftung zwischen der Schicht 7 und den Strukturelementen 8. Die Haftung auf der Schicht 7 wird also erreicht, ohne daß ein Material mit guten Hafteigenschaften für das Metall erforderlich ist.

Im folgenden wird das erfindungsgemäße Verfahren anhand der Figur beschrieben. Es wird mit dem Fachmann bekannten Verfahrensschritten auf dem Halbleitersubstrat 1 die Feldoxidschicht 2 mit der Öffnung 3 und der sich dazwischenbefindende MOS-Transistor 4 ausgebildet. Dabei erfolgt die Herstellung einschließlich der Schicht 7 auf herkömmliche Weise. Die Strukturelemente 8 werden ebenfalls mit in der MOS-Technologie üblichen Verfahrensschritten ausgebildet.

Danach wird gemäß einem ersten Ausführungsbeispiel der Erfindung das Metall für die Metallschicht 9 auf die gesamte Oberfläche der Halbleiteranordnung aufgedampft. Danach muß die so erzeugte Metallschicht selektiv entfernt werden. Dies erfolgt dadurch, daß die Halbleiteranordnung einer Ultraschallbehandlung ausgesetzt wird, bei der sich die Metallschicht von der Oberfläche der Halbleiteranordnung außerhalb der Strukturelemente 8 löst. Vor der Ultraschallbehandlung kann die Halbleiteranordnung mit einem wasserstoffhaltigen Gas behandelt werden. Für die Metallschicht kann Palladium verwendet werden. Da Palladium Wasserstoff aufnimmt, führt diese Behandlung zu einem leichteren Lösen der Metallschicht von der Oberläche der Halbleiteranordnung außerhalb der Strukturelemente 8. Die Hafteigenschaften von Polysilizium der Strukturelemente 8 und dem Paladium sind derart, daß die Paladiumschicht auch während der Ultraschallbehandlung auf dem Silizum haften bleibt. Aufgrund des gewählten Verhältnisses des Abstandes a zwischen den Strukturelementen 8 und der Dicke d der Metallschicht 9 bleibt die Metallschicht auch zwischen den Strukturelementen auf der Schicht 7 haften. Diese Haftung bleibt aufgrund der Geometrie auch dann bestehen, wenn die Schicht 7 aus einem Material mit schlechten Hafteigenschaften für das Metall der Metallschicht 9 ausgebildet ist. Es bleibt dabei insbesondere auch Paladium auf einer Isolatorschicht, die aus Siliziumoxid bestehen kann, haften.

Gemäß einem zweiten Ausführungsbeispiel des erfindungsgemäßen Verfahrens, wird die Metallschicht 9 durch galvanisches, insbesondere durch stromloses galvanisches Abscheiden des Metalls so aufgebracht, daß die Metallschicht 9 nur auf den Strukturelementen 8 und auf der Schicht 7 zwischen den Strukturelementen 8 haften bleibt. Die Haftung auf den Strukturelementen 8 erfolgt hier ebenfalls durch geeignete Wahl des Materials der Strukturelemente 8 und der Metallschicht 9. Die Haftung auf der Schicht 7 zwischen den Strukturelementen 8 wird ebenfalls durch die gewählte Geometrie bedingt. Es ist dabei wesentlich, daß das abzuscheidende Metall ein edleres Metall ist als das Material der Strukturelemente 8.

## Patentansprüche

1. Verfahren zum Herstellen einer Metallelektrode in einer Halbleiteranordnung mit einem MOS-Transistor (4) sowie mit einer die Metallelektrode bildenden, strukturierten Metallschicht (9), die eine Dicke (d) hat, wobei auf der Oberfläche der Halbleiteranordnung in einem Bereich eine Schicht (7) aus einem Material mit schlechten Hafteigenschaften für die Metallschicht (9) aufgebracht wird und auf der Schicht (7) Strukturelemente (8) mit einem Abstand (a) zueinander und aus einem Material mit guten Hafteigenschaften für die Metallschicht (9) ausgebildet werden, wobei die Metallschicht (9) auf die gesamte Oberfläche der Halbleiteranordnung durch Aufdampfen und auschlieβendes selektives Entfernen mittels Ultraschalll oder galvanische Abscheidung aufgebracht wird und auf den Strukturelementen (8) haftet,
**dadurch gekennzeichnet, dass** die Schicht (7) als Gate-Isolationsschicht des MOS-Transistors (4) aufgebracht wird, auf welcher dem Strukturelementen (8) entsprechende Gate-Elektroden mit dem Abstand (α) zueinander angeordnet sind, wobei das Verhältnis der Dicke (d) zum Abstand (a) zwischen den Strukturelementen (8) so gewählt wird, dass die Metallschicht (9) unter Berücksichtigung ihrer Materialeigenschaft nur auf den Strukturelementen (8) und sich von einem zum anderen Strukturelement (8) erstreckend zwischen den Strukturelementen (8) auf der Schicht (7) haften bleibt.

2. Verfahren nach Anspruch 1, **dadurch gekennzeichnet, dass** der Abstand a < 10 µm ist.

3. Verfahren nach einem der vorangehenden Ansprüche, **dadurch gekennzeichnet, dass** zur Metallsierung ein Edelmetall, insbesondere Palladium, verwendet wird.

4. Verfahren nach einem der Ansprüche 1 bis 3, **dadurch gekennzeichnet, dass** die Metallschicht (9) bei dem Aufdampfen und anschlieβenden selektiven Entfernen durch Ultraschall vor dem selektiven Entfernen mit einem wasserstoffhaltigen Gas behandelt wird.

## Claims

1. A method for the manufacture of a metal electrode in a semiconductor configuration with an MOS transistor (4) as well as with a structured metal film (9) forming the metal electrode and having a thickness (d),
whereby a layer (7) made from a material having poor adhesive properties for the metal film (9) is applied to the surface of the semiconductor configuration in one zone and structural elements (8) with a mutual spacing (a) and made from a material having good adhesion properties for the metal film (9) are constructed on the layer (7),
whereby the metal film (9) is applied to the entire surface of the semiconductor configuration by vapour deposition and subsequent selective removal by means of ultrasound or by electrolytic deposition and adheres to the structural elements (8),
**characterised in that** the layer (7) is applied as a gate insulating layer of the MOS transistor (4), on which gate electrodes corresponding to the structural elements (8) are disposed with the mutual spacing (a),
whereby the ratio of the thickness (d) to the spacing (a) between the structural elements (8) is chosen so that the metal film (9), taking into consideration its material properties, remains adhering only to the structural elements (9) and, to the layer (7) extending from one structural element (8) to the other between the structural elements (8).

2. A method according to Claim 1,
**characterised in that** the spacing a is < 10 µm.

3. A method according to one of the preceding Claims,
**characterised in that** a precious metal, in particular palladium, is used for metallisation.

4. A method according to one of Claims 1 to 3,
**characterised in that** during the vapour deposition and subsequent selective removal by ultrasound, the metal film (9) is treated with a hydrogenous gas prior to the selective removal.

## Revendications

1. Procédé de fabrication d'une électrode métallique d'un dispositif semi-conducteur comportant un transistor MOS (4), avec une couche métallique (9) structurée d'une épaisseur (d) et formant l'électrode métallique,
selon lequel
on applique sur la surface du dispositif semi-conducteur, dans une certaine zone, une couche (7) en une matière ayant de mauvaises propriétés d'accrochage pour la couche métallique (9) et sur la couche (7) on développe des éléments de structure (8) ayant une distance (a) les uns par rapport aux autres et en une matière présentant de bonnes propriétés d'accrochage pour la couche métallique (9),
la couche métallique (9) étant appliquée sur toute la surface du dispositif semi-conducteur par dépôt à la vapeur suivi d'enlèvement sélectif par des ultrasons ou par dépôt galvanique et s'accrochant sur les éléments de structure (8),
**caractérisé en ce que**
la couche (7) est appliquée comme couche d'isolation de porte du transistor MOS (4) sur laquelle on a prévu des électrodes de porte écartées de la distance (a) et correspondant aux éléments de structure (8) rapport de l'épaisseur (d) et de la distance (a) entre les éléments de structure (8) est choisi pour qu'en tenant compte des propriétés de la matière, elle reste accrochée entre les éléments de structure (8) et s'étende d'un élément de structure (8) à l'autre.

2. Procédé selon la revendication 1,
**caractérisé en ce que**
la distance (a) est inférieure à 10 µm.

3. Procédé selon l'une des revendications précédentes,
**caractérisé en ce que**
pour la métallisation on utilise un métal noble notamment du palladium.

4. Procédé selon l'une des revendications 1 à 3,
**caractérisé en ce que**
l'on traite la couche métallique (9) par dépôt à la vapeur suivi d'un enlèvement sélectif par ultrasons avant l'enlèvement sélectif avec un gaz contenant de l'hydrogène.
